# NOUVEAU FASCICULE DE BREVET EUROPEEN

(11) **EP 1 807 120 B2**
(45) Date de publication et mention de la décision concernant l'opposition: **10.11.2010**
(45) Mention de la délivrance du brevet: 27.02.2008
(21) Numéro de dépôt: 05800625.5
(22) Date de dépôt: 29.09.2005
(51) Int. Cl.: A61K 8/04, A61K 8/27, A61K 8/29, A61K 8/37, A61Q 1/02, A61Q 1/06, A61Q 1/10, A61Q 1/14, A61Q 17/04, A61Q 19/00

(54) **AGENT MOUILLANT A BASE D'UN MELANGE DE MONOESTERS ET DE DIESTERS DE BUTYLENE GLYCOL**
BENETZUNGSMITTEL AUF BASIS EINER MISCHUNG VON MONOESTERN UND DIESTERN VON BUTYLENGLYCOL
WETTING AGENT BASED ON A MIXTURE OF MONOESTERS AND DIESTERS OF BUTYLENE GLYCOL

(30) Priorité: 05.11.2004 FR 0452547; 08.03.2005 FR 0550604
(43) Date de publication de la demande: 18.07.2007
(73) Titulaire: GATTEFOSSE SAS, 69800 Saint Priest (FR)
(72) Inventeur: RODIER, Jean-David, F-69100 VILLEURBANNE (FR); HUBICHE, Vincent, F-69720 SAINT LAURENT DE MURE (FR)
(74) Mandataire: Denjean, Eric
(86) Numéro de dépôt international: PCT/FR2005/050796
(87) Numéro de publication internationale: WO 2006/048563

(56) Documents cités:
- EP-A2- 1 230 910
- DE-A1- 19 703 471
- DE-A1- 19 956 603
- ANONYMOUS: "AddiActive - Gattefosse: Formulation(s), Soin Réveil JB 1686/A" INTERNET ARTICLE, [Online] 15 juillet 2004 (2004-07-15), XP002328925 Extrait de l'Internet: URL:http://web.archive.org/web/20040715160 926/http://www.addiactive.com/pdffiles/for mulationAA37c.pdf> [extrait le 2005-05-19]

## Description

L'invention concerne un nouvel agent mouillant destiné à être utilisé notamment mais de façon non limitative, dans le domaine de la cosmétique, pour son aptitude à mouiller les pigments. Cette propriété est particulièrement recherchée dans les compositions nécessitant la mise en dispersion des pigments. C'est notamment le cas des produits solaires et des produits de maquillage. Elle est également utile dans les produits de démaquillage pour favoriser la fixation puis l'élimination des pigments présents sur la peau.

De nombreux produits sont proposés pour leur aptitude à mouiller les pigments. On connaît notamment le benzoate d'alkyle en C₁₂-C₁₅ et le palmitate d'isopropyle. Sont également utilisés pour cette même fonction certains triglycérides, tels que, l'huile de ricin ou le tricaprylocaprate de glycérol.

Le document EP-A-792 633 décrit une composition anhydre pulvérulente comprenant un agent liant ou mouillant. En pratique, l'agent liant est un ester, liquide à température ambiante, comprenant au moins deux chaînes hydrocarbonées, comportant chacune au moins dix atomes de carbone. L'ester en question ne présente pas de groupe hydroxyle. Un agent préféré est par exemple le triisostéarate de glycérol.

L'ensemble des agents mouillants connus du Demandeur, tels que précédemment évoqués, présente un certain nombre d'inconvénients. Tout d'abord, la quantité nécessaire de matière première pour mouiller un pigment donné reste relativement élevée. Elle influence la formulation en termes de coût, de composition et de propriétés sensorielles. La forte viscosité de certaines matières premières, telle que l'huile de ricin, à température ambiante ou des dispersions de pigments obtenues en font des produits difficiles à manipuler. Enfin, les agents mouillants connus confèrent à la composition finale dans laquelle ils sont introduits, un toucher parfois gras.

En d'autres termes, il existe un besoin, notamment pour le marché cosmétique, de produits présentant un pouvoir mouillant ne présentant pas les inconvénients ci-dessus.

Dans le cadre de sa recherche, le Demandeur a découvert que les mélanges de monoesters et de diesters de butylène glycol et d'acides gras, caractérisé en ce que l'acide laurique représente au moins 40% en poids des acides gras, dont par exemple le cocoate de butylène glycol, présentaient un pouvoir mouillant plus élevé que celui des agents connus.

Le cocoate de butylène glycol (CBG) est un produit connu, déjà utilisé comme ingrédient cosmétique dans une composition, commercialisée par le Demandeur sous la dénomination EMULFREE^{®} CBG. Plus précisément, cette composition correspond à l'association de CBG avec de l'alcool isostéarylique et de l'éthylcellulose. En pratique, l'EMULFREE^{®} CBG est utilisé comme agent stabilisant de la phase huileuse dans les émulsions huile dans eau. Le rôle du cocoate de butylène glycol au sein de cette composition est cantonné à la fonction de solubilisation du polymère gélifiant, à savoir l'éthylcellulose. En d'autres termes et contrairement à l'invention, le cocoate de butylène glycol n'est pas susceptible d'être mélangé directement avec des charges mais est nécessairement utilisé en combinaison avec un alcool gras (alcool isostéarylique) en présence d'un polymère (éthylcellulose).

Le mélange de l'invention se distingue clairement non seulement en terme de fonction, mais également en terme de structure, des esters de butylène glycol décrits dans le document EP-A-860 164. En effet, ce document décrit l'association d'un filtre UV chimique avec les seuls diesters de butylène glycol avec des acides gras contenant de 6 à 12 atomes de carbone. Non seulement la composition ne contient pas de monoesters, mais en outre elle est utilisée comme agent solubilisant de filtre chimique et non comme agent mouillant.

Le document JP 57072907 décrit un produit démaquillant à base, soit de monoesters, soit de diesters contenant de 17 à 36 atomes de carbone. Là encore, le mélange de monoesters et de diesters n'est pas envisagé. Au surplus, dans le cas des diesters, il s'agit d'esters de diacide, c'est à dire d'un acide estérifié par deux alcools et non d'un diol estérifié par deux acides, comme c'est le cas dans l'invention. Les monoesters sont quant à eux, issus d'un monoacide avec un monoalcool et non d'un monoester d'un diol.

Le document EP-A-651 990 décrit l'utilisation d'esters dans une composition démaquillante. Selon ce document, les esters utilisés sont là encore, soit des esters de monoalcool, soit des diesters. En pratique, ils sont obtenus à partir d'un alcool contenant de 1 à 17 atomes de carbone et d'un acide gras contenant de 3 à 18 atomes de carbone. En outre, ils doivent ne présenter aucun groupement hydroxyle libre.

Le document EP-A- 230 910 décrit une émulsion huile dans eau contenant des pigments et dont la phase huileuse contient les seuls diesters caprylocaprate de butylène glycol.

En d'autres termes, rien dans l'état de la technique connu du Demandeur ne divulgue l'idée d'utiliser un mélange de monoesters et de diesters de butylène glycol, comme agent mouillant.

Dès lors, l'invention concerne l'utilisation comme agent mouillant de pigments d'un mélange de monoesters et de diesters de butylène glycol et d'acides gras caractérisé en ce que l'acide laurique représente au moins 40 % en poids des acides gras.

Selon une première caractéristique, les acides gras sont choisis dans le groupe comprenant les acides gras en C₆ à C₂₂. Bien entendu, les monoesters et diesters peuvent être obtenus à partir d'acides gras différents et/ou identiques, saturés et/ou insaturés.

Dans un mode de réalisation particulier, l'agent mouillant de l'invention est obtenu par estérification de butylène glycol avec tout ou partie d'acides gras d'origine naturelle, en particulier issus d'huile végétale.

Le Demandeur a constaté qu'on obtenait de bons résultats lorsque l'acide laurique représentait au moins 40 %, avantageusement de 50 à 60% en poids des acides gras. On retrouve une telle répartition dans l'huile de coprah ou de palmiste.

Dès lors et selon un mode de réalisation préféré, l'agent mouillant est obtenu par estérification de butylène glycol et d'une fraction d'acides gras en C₁₂ à C₁₈ issue de l'huile de coprah.

Selon une autre caractéristique, les monoesters représentent entre 10 et 90%, avantageusement entre 40 et 60% en poids du mélange monoesters/diesters.

L'agent mouillant décrit présente un intérêt technologique car il facilite le procédé de formulation. En effet, il s'emploie en proportion plus faible pour une quantité de pigments donnée par rapport aux mouillants classiques. Il procure une viscosité des dispersions plus faible. Elles sont donc plus faciles à employer que celles obtenues avec des mouillants classiques. Enfin, la quantité moins importante de mouillant de l'invention à utiliser permet de laisser place à des agents de texture ou de toucher incorporables dans la formule. En outre, cet agent mouillant procure un toucher sec non gras.

Comme déjà dit, ce produit peut être utilisé dans toutes les compositions cosmétiques ou dermatologiques comprenant des pigments permettant ainsi, de part ses propriétés mouillantes, d'en améliorer la dispersion dans la phase huileuse, et d'optimiser l'étalement de la composition sur la peau. Bien entendu, la proportion d'agent mouillant dans la composition sera fonction de la nature et de la quantité des pigments.

En particulier, l'utilisation de l'agent de l'invention sera plus particulièrement intéressante dans les produits contenant des pigments tels que produits solaires, produits de maquillage (fond de teint, mascara, rouge à lèvres, etc..), crèmes pour bébé incorporant des pigments ou les produits destinés à éliminer les pigments tels que les produits de démaquillage.

Les compositions dans lesquelles on peut introduire l'agent mouillant de l'invention peuvent se présenter sous la forme d'émulsion eau dans huile, huile dans eau, multiple, ou sous forme anhydre.

Elles peuvent notamment contenir les adjuvants habituels dans les domaines cosmétique et dermatologique, tels que les matières grasses, les émulsionnants et co-émulsionnants, les gélifiants hydrophiles ou lipophiles, les actifs hydrophiles ou lipophiles, les conservateurs, les antioxydants, les solvants, les parfums, les charges, les filtres hydrophiles et lipophiles, les matières colorantes, les neutralisants, les agents pro-pénétrants, et les polymères.

Les quantités de ces différents adjuvants sont celles classiquement utilisées dans les domaines considérés, et par exemple de 0.01 à 30% du poids total de la composition. Ces adjuvants, selon leur nature, peuvent être introduits dans la phase grasse ou dans la phase aqueuse.

Comme matières grasses utilisables, on peut utiliser les huiles minérales, les huiles d'origine animale (lanoline), les huiles de synthèse (isopropyl myristate, octyldodecyl, isostearyl isostearate, decyl oleate, isopropyl palmitate), les huiles siliconées (cyclomethicone, dimethicone) et les huiles fluorées. On peut utiliser comme matières grasses des alcools gras, des acides gras, des cires et des gommes et en particulier les gommes de silicone.

Comme émulsionnants et co-émulsionnants utilisables, on peut citer par exemple les esters de polyglycérols et d'acides gras, les esters de sucrose et d'acides gras, les esters de sorbitane et d'acides gras, les esters d'acides gras et de sorbitane oxyéthylénés, les ethers d'alcools gras et de PEG, les esters de glycerol et d'acides gras, les alkyl sulfates, les alkyl ether sulfates, les alkyl phosphates, les alkyl polyglucosides, les dimethicone copolyols.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides tels que la gomme xanthane, la gomme guar, les gommes naturelles telles que la gomme de cellulose et dérivés, et les argiles.

Comme gélifiants lipophiles, on peut citer les argiles modifiées comme les bentones, les sels métalliques d'acides gras, la silice hydrophobe et l'éthylcellulose.

Comme actifs, on peut utiliser notamment les dépigmentants, les émollients, les hydratants, les anti-séborrhéiques, les anti-acnéiques, les agents kératolytiques et/ou desquamants, les agents anti-rides et tenseurs, les agents draînants, les agents anti-irritants, les agents apaisants, les amincissants tels que les bases xanthiques (caféine), les vitamines et leurs mélanges.

Comme conservateurs utilisables, on peut citer l'acide benzoïque, ses sels et ses esters ; l'acide sorbique et ses sels ; les parabens, leurs sels et esters ; le triclosan ; l'imidazolidinyl urée ; le phenoxyethanol ; la DMDM hydantoïne ; le diazolidinyl urée ; la chlorphenesin.

Comme antioxydants utilisables, on peut citer les agents chelatants tels que l'EDTA et ses sels.

Comme solvants utilisables, on peut citer l'eau, l'éthanol, la glycérine, le propylène glycol, le butylène glycol, le sorbitol.

Comme charges utilisables, on peut citer le talc, le kaolin, le mica, la sérécite, le magnésium carbonate, l'aluminium silicate, le magnésium silicate, les poudres organiques telles que le nylon.

Comme filtres utilisables, on peut citer les filtres UVA et UVB classiquement utilisés tels que la benzophenone-3, le butyl methoxydibenzoyl methane, l'octocrylène, l'octyl methoxycinnamate, le 4-methylbenzylidene camphor, l'octyl salicylate.

Comme matières colorantes utilisables, on peut citer les colorants lipophiles, les colorants hydrophiles, et les nacres habituellement utilisés dans les compositions cosmétiques ou dermatologiques, et leurs mélanges.

Comme neutralisants utilisables, on peut citer la soude, la triethanolamine, l'aminomethyl propanol, l'hydroxyde de potassium. »

Il est par ailleurs également possible de proposer des formulations correspondant alors à des dispersions à base uniquement de pigments et de l'agent mouillant de l'invention. On peut incorporer un agent stabilisant de type polymérique qui permet d'éviter la sédimentation des pigments. La dispersion restera lisse et homogène. Cette dispersion peut également contenir des phases lipophiles de type huiles ou esters. Cette dispersion peut ensuite être incorporée telle quelle dans la composition cosmétique plus complexe.

L'invention et les avantages qui en découlent ressortiront bien des exemples de réalisation suivant, à l'appui des figures annexées.

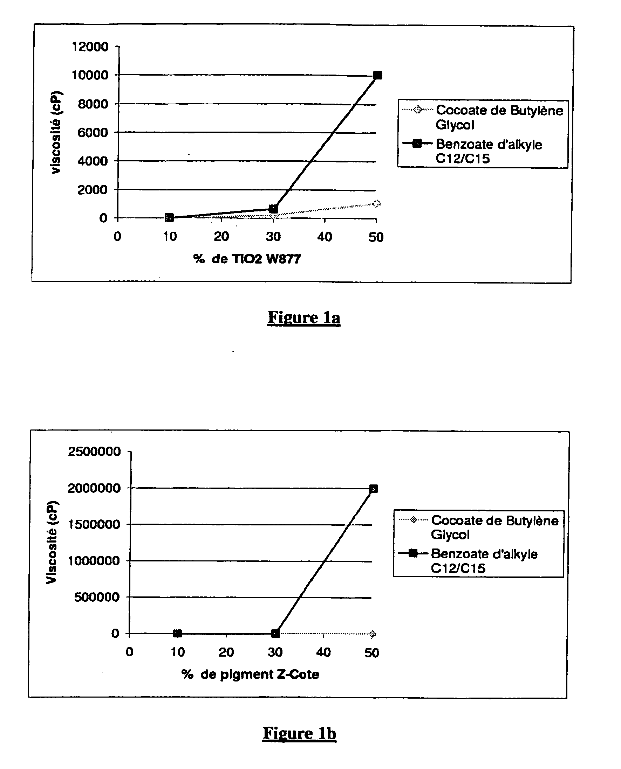

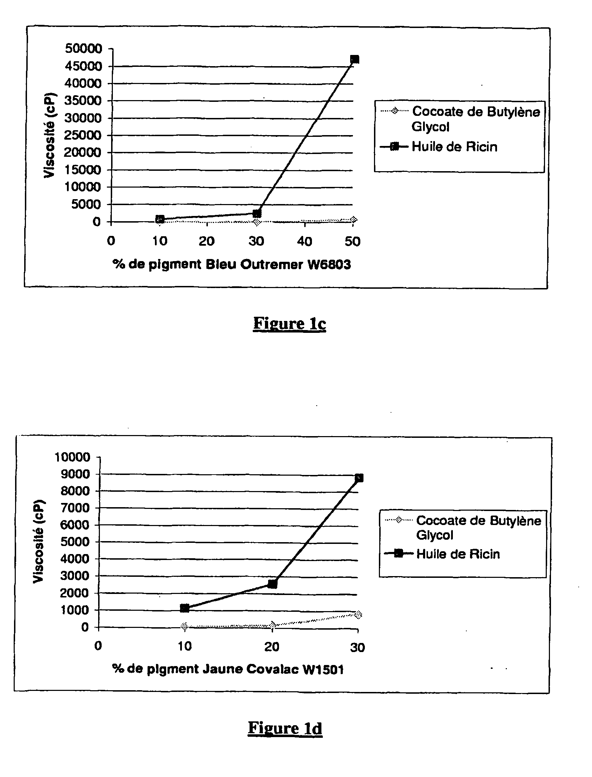

Les figures 1a, b, c, d, sont des courbes représentant la viscosité d'une dispersion formulée avec l'agent mouillant de l'invention ou un agent de l'art antérieur (benzoate d'alkyle en C₁₂ à C₁₅ ou huile de ricin) en fonction d'une quantité croissante de pigments.

Les figures 2 sont des photographies prises au microscope optique (grossissement x 400) d'une émulsion pigmentée formulée avec du CBG (figure 2A) ou avec du benzoate d'alkyle en C₁₂ à C₁₅ (figure 2B).

### Exemple 1 : Pouvoir mouillant et viscosité par rapport à divers agents mouillants

### 1. Evaluation du pouvoir mouillant

Le pouvoir mouillant d'une huile ou d'un solvant est la quantité minimale d'huile ou de solvant nécessaire pour former avec une poudre, une pâte lisse sans craquèlement lors de son étalement.

On l'exprime en gramme d'huile nécessaire pour mouiller 100g de pigment. Plus la quantité d'huile à utiliser est faible, meilleur est le pouvoir mouillant.

### 2. Résultats du pouvoir mouillant par rapport aux mouillants classiques

### 2.a Pouvoir mouillant

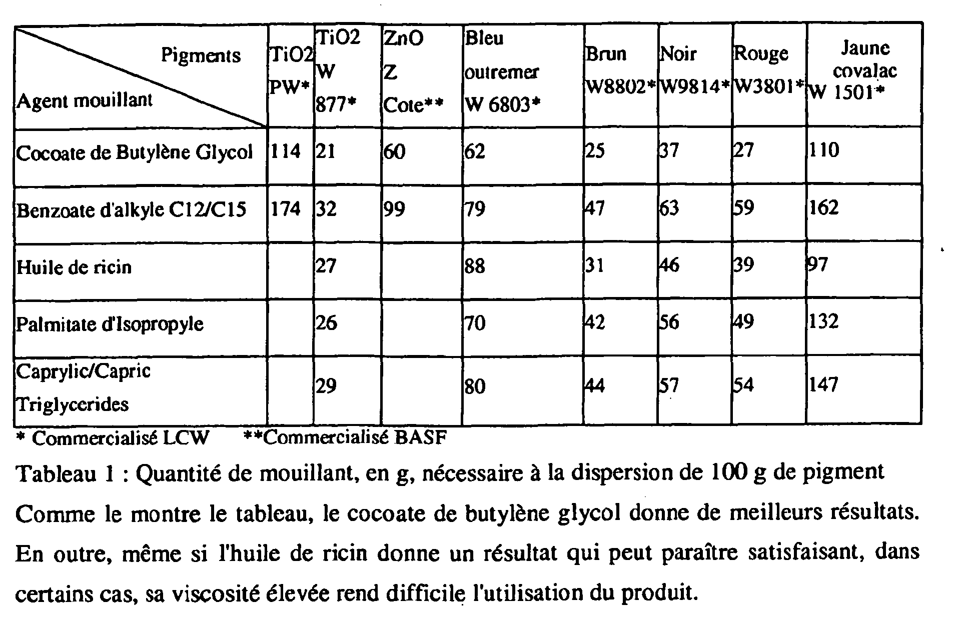

### 2.b.Viscosité des dispersions

Les figures la, b, c, d, sont des courbes représentant la viscosité d'une dispersion formulée avec l'agent mouillant de l'invention ou un agent de l'art antérieur (benzoate d'alkyle en C₁₂ à C₁₅ ou huile de ricin) en fonction d'une quantité croissante de pigments. Les dispersions obtenues avec le CBG sont plus fluides qu'avec le benzoate d'alkyle en C₁₂ à C₁₅ ou l'huile de ricin. Ainsi pour une même composition, une dispersion dans le CBG sera plus facile d'emploi.

### 3 Résultats du pouvoir mouillant par rapport à un diester de butylène glycol

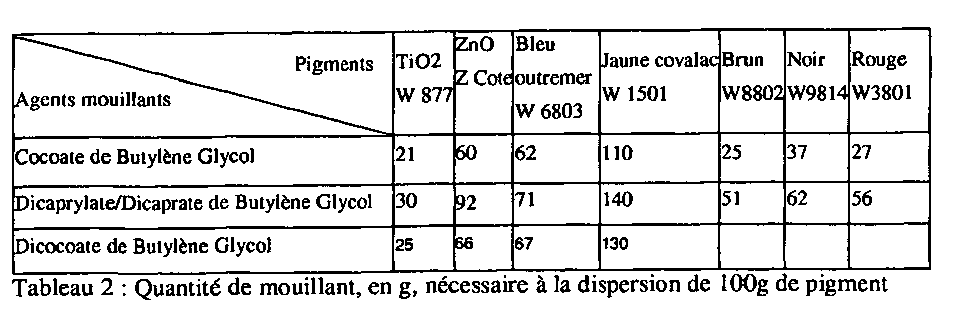

Comme le montre le tableau, l'agent mouillant de l'invention est plus efficace que le produit décrit dans l'art antérieur (EP-A-1 230 910), utilisé comme solubilisant de filtre.

Conclusion : le produit de l'invention mélange de monoester et de diester est plus efficace qu'un diester.

### 4 Résultat du pouvoir mouillant par rapport aux esters de glycol (monoesters et diesters)

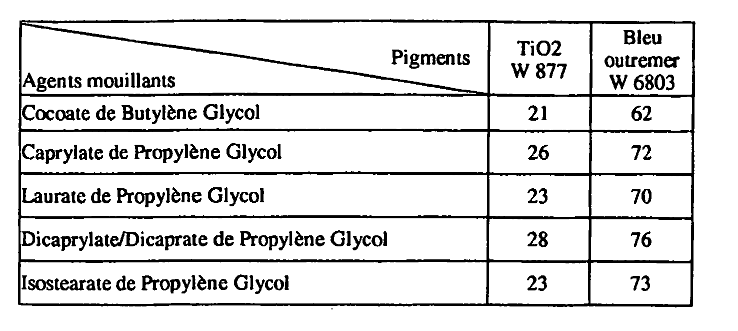

Conclusion : Quelle que soit la nature de la longueur de la chaîne grasse (donnant un produit liquide), le CBG donne un meilleur résultat que les esters de propylène glycol (diesters ou mélange de mono/diesters) ou que les diesters de butylène glycol.

### Exemple 2 : Evaluation en formulation. Mesure du facteur de protection solaire (FPS)

L'excellent pouvoir mouillant démontré pour le CBG trouve une application intéressante dans les produits solaires contenant des filtres minéraux tels que le dioxyde de titane et l'oxyde de zinc. En effet un meilleur mouillage de ces pigments en formulation permet une meilleure dispersion et un meilleur étalement de ceux-ci sur la peau, donc une meilleure protection solaire.

Deux émulsions huile dans eau ont été formulées avec respectivement du CBG (A) et du benzoate d'alkyle en C₁₂-C₁₅ (B).

| | | A | B |
|---|---|---|---|
| Phase I | Emulium® 22 | 4 | 4 |
| | Huile minérale | 2 | 2 |
| | Dimethicone | 2 | 2 |
| | Cocoate de Butylène Glycol | 23 | - |
| | C12-C15 alkyl benzoate | - | 23 |
| | Dioxyde de Titane | 10 | 10 |
| | Oxyde de Zinc | 5 | 5 |
| | Conservateur | qs | qs |
| Phase II | Eau déminéralisée | Qsp 100 | Qsp 100 |
| | Gomme xanthane | 0.4 | 0.4 |
| | Cellulose microcrystalline et gomme de cellulose | 1 | 1 |
| | Glycérine | 3 | 3 |

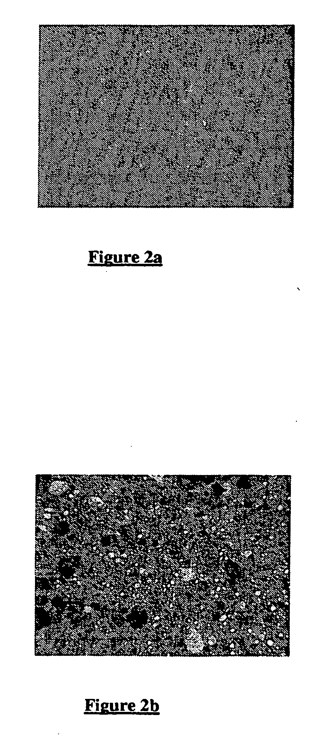

Les figures 2 sont des photographies prises au microscope optique (grossissement x 400) d'une émulsion pigmentée formulée avec du CBG (figure 2A) ou avec du benzoate d'alkyle en C₁₂ à C₁₅ (figure 2B).

On note une grande différence dans la dispersion des pigments. En effet dans la formule A contenant le CBG (figure 2A), les pigments sont bien dispersés, alors que dans la formule B contenant le benzoate d'alkyle en C₁₂-C₁₅ (figure 2B) les pigments apparaissent sous forme d'agrégats.

Les facteurs de protection solaire ont été mesurés in vivo selon les recommandations du COLIPA sur 5 volontaires :

| Formule A | Formule B |
|---|---|
| 11.5+/-2.9 | 8.7+/-2.8 |

Ces résultats montrent de façon significative (p<0.05) que l'utilisation du CBG à la place du benzoate d'alkyle en C₁₂-C₁₅ permet d'obtenir un facteur de protection solaire (FPS) plus élevé.

D'autre part au niveau sensoriel, la formule A est significativement plus facile à étaler sur la peau que la formule B.

### Exemple 3 : Formules

Figurent ci-après quelques exemples de formules réalisables avec les dispersions de pigments contenant le CBG dans plusieurs applications.

### 1. Solaire

Crème solaire eau dans huile :

| | Ingrédients | % m/m |
|---|---|---|
| Phase I | Plurol® Diisostéarique | 5 |
| | Cocoate de Butylène Glycol | 10 |
| | Isohexadecane | 10 |
| | Dimethicone et dimethiconol | 2 |
| | Dioxyde de Titane | 10 |
| | Conservateur | qs |
| Phase II | Eau déminéralisée | Qsp 100 |
| | MgSO4, 7H2O | 0.5 |
| | NaCl | 0.5 |
| | Glycérine | 3 |

Le FPS de la formule est mesuré à 21 *in vivo.*

| | Ingrédients | % m/m |
|---|---|---|
| Phase I | Cetyl Dimethicone Copolyol | 3.50 |
| | Diisostearyl Polyglyceryl-3 Dimer Dilinoleate | 1.50 |
| | Octyl Methoxycinnamate | 7.50 |
| | Octocrylene | 2.00 |
| | Cyclomethicone | 8.00 |
| | Acetate de Tocopherol | 0.50 |
| | Conservateur | Qs |
| Phase II | Cocoate de Butylène Glycol | 10.00 |
| | Dioxide de Titane & Trimethoxycaprylylsilane | 10.00 |
| Phase III | Eau déminéralisée | Qsp 100 |
| | NaCl | 0.50 |
| | Glycérine | 3.00 |

Le Facteur de Protection Solaire (FPS) de la formule est mesuré à 43.6 *in vivo.*

### 2. Maquillage

Fond de teint

| | Ingrédients | % m/m |
|---|---|---|
| Phase I | Hydrolactol® 70 | 5 |
| | Alcool cétostéarylique | 2 |
| | Alcool cétylique | 1 |
| | Dimethicone | 4 |
| | Cyclomethicone | 2 |
| | Isostéarate d'isostéaryle | 5 |
| | Conservateur | qs |
| Phase II | Eau déminéralisée | Qsp 100 |
| | Cellulose microcrystalline et gomme de cellulose | 1.5 |
| | Gomme de xanthane | 0.4 |
| | EDTA, Na2 | 0.1 |
| Phase III | Cocoate de Butylène Glycol | 3 |
| | Dioxyde de Titane | 6.6 |
| | Oxyde de Fer jaune | 1.2 |
| | Pigment Brun | 0.25 |
| | Oxyde de Fer rouge | 0.53 |
| | Oxyde de Fer noir | 0.11 |

### Mascara

| | Ingrédients | % m/m |
|---|---|---|
| Phase I | Apifil® | 8 |
| | Compritol® 888 | 2 |
| | Dimethicone | 2 |
| Phase II | Eau déminéralisée | Qsp 100 |
| | Carbomer | 0.2 |
| Phase III | Soude à 10% | 0.4 |
| Phase IV | Cocoate de Butylène Glycol | 8 |
| | Oxyde de Fer noir | 12 |
| | Talc | 4 |
| Phase V | PVP/Dimethylaminoethyl methacrylate copolymer | 10 |
| | Conservateur | Qs |

### 3. Rouge à lèvre

| Ingrédients | % m/m |
|---|---|
| Base de rouge à lèvre CB8047 (Gattefossé) | 73.35 |
| Cire de Candelilla | 4 |
| Ozokerite | 2 |
| Ethoxydiglycol Behenate | 8 |
| Cocoate de Butylène Glycol | 8 |
| Jaune Covalac W1501 | 2 |
| Ruby Covanor W4605 | 1 |
| Dioxyde de Titane W877 | 1.65 |

### 4. Démaquillage

Lait démaquillant

| | Ingrédients | % m/m |
|---|---|---|
| Phase I | Eau déminéralisée | Qsp 100 |
| | Polymère acrylique | 0.3 |
| | Gomme xanthane | 0.2 |
| | Conservateur | qs |
| Phase II | Cocoate de Butylène Glycol | 10 |
| | Cyclomethicone | 2 |
| Phase III | Soude à 10% | 0.6 |

### 5. Produits pour bébé

Crème pour bébé avec oxyde de zinc

| | Ingrédients | % m/m |
|---|---|---|
| Phase I | Plurol® Diisostéarique | 5 |
| | Huile minérale | 15 |
| | Cocoate de Butylène Glycol | 7 |
| | Oxyde de Zinc | 15 |
| | Compritol® 888 | 5 |
| | Conservateur | Qs |
| Phase II | Eau déminéralisée | Qsp 100 |
| | NaCl | 0.5 |
| | MgSO4,7H2O | 0.5 |
| Phase III | Extrait de Calendula officinalis | 2 |
| | Parfum | qs |

## Revendications

1. Utilisation comme agent mouillant de pigments d'un mélange de monoesters et de diesters de butylène glycol et d'acides gras, **caractérisé en ce que** l'acide laurique représente au moins 40 % en poids des acides gras.

2. Utilisation selon la revendication 1, **caractérisé en ce que** l'acide laurique représente entre 50 et 60% en poids des acides gras.

3. Utilisation selon l'une des revendications précédentes, **caractérisé en ce qu'**il est obtenu par estérification de butylène glycol avec une fraction d'acides gras en C₁₂ à C₁₈ issue d'huile de coprah.

4. Utilisation selon l'une des revendications précédentes, **caractérisé en ce que** les monoesters représentent entre 10 et 90%, avantageusement entre 40 et 60% en poids du mélange monoesters/diesters.

5. Dispersion constituée du mélange de monoesters et de diesters de butylène glycol et d'acides gras mentionné dans l'une des revendications 1 à 4, et de pigments.

6. Dispersion constituée du mélange de monoesters et de diesters de butylène glycol et d'acides gras mentionné dans l'une des revendications 1 à 4, de pigments et d'un agent stabilisant.

7. Dispersion constituée du mélange de monoesters et de diesters de butylène glycol et d'acides gras mentionné dans l'une des revendications 1 à 4, de pigments, d'au moins une phase lipophile et éventuellement d'un agent stabilisant.

## Claims

1. Use of a mixture of monoesters and diesters of butylene glycol and fatty acids as pigment wetting agent, **characterized in that** lauric acid accounts for at least 40% by weight of the fatty acids

2. Use according to Claim 1, **characterized in that** lauric acid accounts for between 50 and 60% by weight of the fatty acids.

3. Use according to one of the preceding claims, **characterized in that** it is obtained by esterification of butylene glycol with a fraction of C₁₂ to C₁₈ fatty acids issuing from copra oil.

4. Use according to one of the preceding claims, **characterized in that** the monoesters account for between 10 and 90%, advantageously between 40 and 60% by weight of the monoester/diester mixture.

5. Dispersion consisting of a mixture of monoesters and diesters of butylene glycol and fatty acids mentioned in one of Claims 1 to 4, and of pigments.

6. Dispersion consisting of a mixture of monoesters and diesters of butylene glycol and fatty acids mentioned in one of Claims 1 to 4, pigments and a stabilizer.

7. Dispersion consisting of the mixture of monoesters and diesters of butylene glycol and fatty acids mentioned in one of Claims 1 to 4, pigments, at least one lipophilic phase and, optionally, a stabilizer.

## Patentansprüche

1. Verwendung einer Mischung von Monoestern und Diestern von Butylenglycol und Fettsäuren als Benetzungsmittel von Pigmenten, **dadurch gekennzeichnet, dass** die Laurinsäure mindestens 40 % vom Gewicht der Fettsäuren darstellt.

2. Verwendung nach Anspruch 1, **dadurch gekennzeichnet, dass** die Laurinsäure 50 bis 60 % vom Gewicht der Fettsäuren darstellt.

3. Verwendung nach einem der vorhergehenden Ansprüche, **dadurch gekennzeichnet, dass** es durch Veresterung von Butylenglycol mit einer von Koprahöl stammenden (C₁₂-C₁₈)-Fettsäurenfraktion hergestellt ist.

4. Verwendung nach einem der vorhergehenden Ansprüche, **dadurch gekennzeichnet, dass** die Monoester zwischen 10 und 90 %, vorteilhafterweise zwischen 40 und 60 % vom Gewicht der Mischung von Monoestern/Diestern darstellen.

5. Dispersion, die aus der in einem der Ansprüche 1 bis 4 erwähnten Mischung von Monoestern und Diestern von Butylenglycol und Fettsäuren und aus Pigmenten besteht.

6. Dispersion, die aus der in einem der Ansprüche 1 bis 4 erwähnten Mischung von Monoestern und Diestern von Butylenglycol und Fettsäuren, aus Pigmenten und einem Stabilisator besteht.

7. Dispersion, die aus der in einem der Ansprüche 1 bis 4 erwähnten Mischung von Monoestern und Diestern von Butylenglycol und Fettsäuren, aus Pigmenten, mindestens einer lipophilen Phase und gegebenenfalls einem Stabilisator besteht.
